# EUROPEAN PATENT APPLICATION

(11) **EP 4 450 062 A1**
(43) Date of publication of application: **23.10.2024**
(21) Application number: 23169052.0
(22) Date of filing: 20.04.2023
(51) Int. Cl.: A61K 9/00, A61K 47/12, A61K 47/36, A61K 31/663

(54) **COMPOSITION FOR THE TREATMENT OF OSTEOARTHROSIS**

(71) Applicant: Abiogen Pharma S.p.A., 56121 Pisa (IT)
(72) Inventor: FONTANAZZA, Giulia, 69630 Chaponost (FR); MOUSEL, Caroline Simone, 69630 Chaponost (FR); FUCHEZ, Fabien, 69630 Chaponost (FR)
(74) Representative: Cattaneo, Elisabetta

(57) **Abstract**

The invention concerns a composition for intra-articular use for the treatment of osteoarthrosis (OA) comprising crosslinked hyaluronic acid or a salt or derivate thereof, clodronic acid or equivalent amounts of a salt thereof, and acetate buffer.

## Description

### FIELD OF THE INVENTION

The invention concerns a composition, preferably for use in the treatment of osteoarthrosis (OA), also including osteoarthritis, through intra-articular administration, comprising crosslinked hyaluronic acid or a salt or derivate thereof, clodronic acid or equivalent amounts of a salt thereof, and acetate buffer.

### TECHNICAL FIELD

Arthrosis or osteoarthrosis (OA) is a progressive chronic illness affecting particularly the joints most subjected to mechanical stresses, such as hips and knees. When this condition arises, the whole joint is affected by a series of degradation and repair processes which eventually alter the anatomy and function of the joint, affecting all the joint components such as the cartilage, subchondral bone, and synovial tissues. The "arthrosic" condition is therefore the result of a set of interrelations between systemic factors (*e.g*. advanced age or obesity) and local factors (*e.g*. trauma or excessive use) which are in turn modulated by numerous predisposing factors, possibly combined with infectious and inflammatory events with various aetiologies. In this complexity of events, attempts have been made over time to therapeutically intervene by addressing and counteracting the individual causal processes, and resorting to a variety of different therapeutic approaches, essentially oriented towards the management of inflammatory events and the pain caused, mainly using analgesics, local or systemic anti-inflammatories, or intra-articular injections of various drugs such as, for example, bisphosphonates, hyaluronic acid, anthraquinones, chondroitin sulfate.

Combinations of the above active ingredients have also been attempted in order to obtain even more effective compositions.

For example, in the patent EP1976538, owned by the same Applicant, a combination of linear hyaluronic acid with sodium clodronate is described, which allows to obtain excellent results in terms of reduction of the painful symptoms in patients affected by OA. Furthermore, the combination of the two active ingredients, by virtue of their synergistic effect, allows to reduce in the final formulation both the effective amount of hyaluronic acid used, reducing its undesirable effects such as the stimulation of the production of inflammatory cytokines, and the amount of clodronate, reducing adverse effects, mainly due to its acidity, thus improving overall patient compliance.

After tens of years of clinical use of hyaluronic acid, it is now known that the effectiveness of linear hyaluronic acid is well exceeded by crosslinked hyaluronic acid, the latter being characterized by a longer residence time in the joint compared to linear hyaluronic.

Scientific studies show a residence time of about 1-4 weeks compared to only about 2-3 days for linear hyaluronic acid (Conrozier, Joint Bone Spine 83, 2016, 1-2). The literature reposts that this is due to its particular chemical structure, which makes it less vulnerable to degradation by hyaluronidases naturally present inside the joint cavity.

It is also known that the association of a crosslinked hyaluronic acid with clodronic acid or salts thereof, for example the sodium salt, has therapeutic efficacy. Palmieri, Drug Design, Development and Therapy 2013:7 7-12, for example, reports the positive results of the use of the two active ingredients for the treatment of painful symptoms of osteoarthrosis in the knee; in this study the authors mixed solutions of commercial drugs, containing the separate active ingredients, just before use, to obtain an extemporaneous solution of their combination.

However, the drug thus made, *i.e*. extemporaneously, must be used quickly because the two active ingredients are not very stable once placed in contact with each other, and the drug tends to degrade quickly.

*De facto,* even today, the main therapeutic objective in the treatment of OA is therefore the management and reduction of the painful symptoms that always accompany this pathology, and which make the quality of life of the affected patients critical. This objective is also to be achieved through therapeutic approaches that are as least invasive as possible.

The object of the present invention is therefore to be able to solve the problems reported above by obtaining a new therapeutic alternative for the treatment of OA, and, in particular, for the treatment of the painful symptoms of OA, which is at the same time effective, safe and with a satisfactory patient compliance.

### SUMMARY

The inventors of the present invention have surprisingly found that it was possible to obtain said valid therapeutic alternative by using crosslinked hyaluronic acid or a salt or derivative thereof, clodronic acid or equivalent amounts of a salt thereof, in combination with acetate buffer.

In fact, the addition of acetate buffer to a composition comprising crosslinked hyaluronic acid or a salt or derivative thereof, and clodronic acid or equivalent amounts of a salt thereof, has enabled the composition to prove to be surprisingly stable, with excellent compliance while being effective and safe for the patient. Surprisingly, the presence of the acetate buffer was in fact able to stabilize the composition of crosslinked hyaluronic acid, or a salt or derivative thereof, and clodronic acid, or equivalent amounts of a salt thereof, so effectively that it was possible to also overcome the stability issues connected to the necessary sterilization treatments of the pharmaceutical product during the drug manufacturing phase, which in the absence of acetate buffer, or using other buffers, led instead to rapid degradation of the solution.

The invention therefore concerns a composition comprising:
- crosslinked hyaluronic acid or a salt or derivate thereof,
- clodronic acid or equivalent amounts of a salt thereof, and
- acetate buffer.

In the present invention, when the term "acetate buffer" is used, it is intended to mean an aqueous solution of acetic acid and an alkali or alkaline earth metal acetate. Examples of acetate buffer include aqueous solutions of acetic acid-sodium acetate, acetic acid-potassium acetate, acetic acid-calcium acetate, acetic acid-magnesium acetate, and the like. The preferred acetate buffer according to the invention is an aqueous solution of acetic acid-sodium acetate.

The composition of the invention optionally comprises a pharmaceutically acceptable carrier and/or suitable excipients.

In a preferred embodiment, the composition of the invention is in the form of a gel. In a more preferred embodiment said composition of the invention, preferably in the form of a gel, comprises:
- from 1.8 to 2.2 mg/mL, preferably from 1.9 to 2.1 mg/mL, more preferably about 2 mg/mL of sodium clodronate or equivalent amounts of clodronic acid or other salt thereof;
- from 16 to 24 mg/mL, preferably from 18 to 22 mg/mL, more preferably about 20 mg/mL of crosslinked sodium hyaluronate;
- from 0.6 to 1.2 mg/mL, preferably from 0.8 to 1 mg/mL, of sodium acetate and from 0.028 to 0.052 mg/mL of acetic acid, and
- water

The composition of the invention in the form of a gel optionally comprises excipients. In a further preferred embodiment, said composition, preferably in the form of a gel, comprises:
- from 1.8 to 2.2 mg/mL, preferably from 1.9 to 2.1 mg/mL, more preferably about 2 mg/mL of sodium clodronate or equivalent amounts of clodronic acid or other salt thereof;
- from 17 to 21 mg/mL, preferably from 18 to 20 mg/mL, more preferably 19 mg/mL of crosslinked sodium hyaluronate;
- from 0.6 to 1.2 mg/mL, preferably from 0.8 to 1 mg/mL, of sodium acetate and from 0.028 to 0.052 mg/mL of acetic acid;
- from 0.5 to 2 mg/mL, preferably about 1 mg/mL of linear sodium hyaluronate, and
- Water.

Optionally, the composition of the invention further comprises excipients.

In another aspect, the invention concerns a single-dose administration form comprising from 1 to 5 mL, preferably 2 mL, of the above composition in the form of a gel.

In a further aspect, the invention concerns the composition of the invention for use alone or in combination, *i.e*. as a co-therapeutic agent in a fixed dose combination or combination therapy with separately formulated active ingredients, in the treatment of osteoarthrosis through intra-articular administration.

In the present invention, when "osteoarthrosis" is indicated, it is also intended to include "osteoarthritis".

The inventors also realized that the composition of the invention made it possible to improve the symptoms of osteoarthrosis.

The invention therefore concerns a medical device comprising the composition of the invention and excipients.

Said medical device, and therefore the composition of the invention, can be used to improve the symptoms of osteoarthrosis, in particular in relation to the aspects relating to joint pain.

Said medical device and therefore the composition of the invention can then be used in a combination therapy with a known drug for the treatment of osteoarthrosis.

In another aspect, the present invention concerns a process for the preparation of the above composition.

### DETAILED DESCRIPTION OF THE INVENTION

In the present invention the following terms:
- "clodronic acid or equivalent amounts of a salt thereof' is intended to include all the polymorphic forms thereof, both amorphous and crystalline, and co-crystalline, as well as anhydrous, hydrated and solvate forms of clodronic acid or salts thereof. The salt is intended to be formed with any pharmaceutically acceptable cation; the preferred cation is sodium.
- "acetate buffer" means an aqueous solution of acetic acid and alkali or alkaline earth metal acetate. Examples of acetate buffer include aqueous solutions of acetic acid-sodium acetate, acetic acid-potassium acetate, acetic acid-calcium acetate, acetic acid-magnesium acetate, and the like. The preferred acetate buffer according to the invention is an aqueous solution of acetic acid-sodium acetate.
- "crosslinked hyaluronic acid or a salt or derivative thereof' means hyaluronic acid crosslinked, for example, with divinylsulfone (DVS), glutaraldehyde (GTA), 1,4-butanediol diglycidyl ether (BDDE) or other chemical crosslinking methods, or crosslinked by physical crosslinking methods (for example photo-crosslinking or thermo-crosslinking), or a salt thereof with any pharmaceutically acceptable cation; the preferred cation is sodium; or derivatized hyaluronic acid, for example, with a compound selected from the group consisting of carbodiimides, carbonyldiimidazole, hydrazine sulfate; the derivatives also include the organic and inorganic salts of crosslinked or derivatized hyaluronic acids with the methods described above. The preferred crosslinked hyaluronic acid according to the invention is crosslinked hyaluronic acid obtained by crosslinking with 1,4-butanediol diglycidyl ether (BDDE).
- "linear hyaluronic acid or salt thereof' means hyaluronic acid not subjected to any crosslinking process and the salt thereof is intended with any pharmaceutically acceptable cation; the preferred cation is sodium.
- "pharmaceutically acceptable carrier" means a carrier suitable for the final formulation, preferably this carrier is water, more preferably water for intra-articular administration.
- "Osteoarthrosis (OA)" means a degenerative and/or inflammatory process affecting the joints, including osteoarthritis.
- G' or elastic modulus means the energy stored in the elastic structure of a solid (for example a gel) during deformation; the higher the G' the greater the hardness of the material. The elastic modulus G' is conventionally used to describe the solid component of a gel.

In the context of the present invention, the modulus G' is preferably determined at room temperature, typically at a temperature of 25°C, with the aid of a rheometer, in this case a Kinexus Pro type, using a plate/plate geometry of 40 mm and a gap of 250 µm.

A pre-shearing and a rest period are applied to the sample to erase the "rheological memory", then the linear viscoelastic domain is determined by amplitude sweep, for example at a fixed frequency of 1 Hz.

The modulus G' can then be determined from the curve obtained in the linear viscoelastic region where stress and displacement are in a linear relationship.
- The term "extrusion force" means the force required to push the product out of the syringe through the needle.

In the present invention, the extrusion force is measured by means of a universal testing machine (or universal tester machine) of the MTS Adamel Lhomargy DY30 type.

According to the protocol used, the needle is connected to the syringe, a speed of 1 cm/minute is applied to the plunger of the syringe, and the average extrusion force between 10 mm and 30 mm of displacement is recorded.

In the context of the present description and in the subsequent claims, all the numerical quantities indicating amounts, parameters, percentages, and so on, are to be intended in all circumstances as preceded by the term "about" unless otherwise indicated. Furthermore, all numerical quantities ranges include all possible combinations of maximum and minimum numerical values and all possible intermediate ranges, in addition to those specifically indicated in the following description. Preferably, the composition for use in the treatment of osteoarthrosis (OA) of the invention is directed to the treatment of osteoarthrosis (OA) painful symptoms.

The invention therefore concerns a composition comprising:
- crosslinked hyaluronic acid or a salt or derivative thereof,
- clodronic acid, or equivalent amounts of a salt thereof, and
- acetate buffer.

The composition of the invention therefore comprises clodronic acid or equivalent amounts of a salt thereof. Salts of clodronic acid are preferably salts of clodronic acid with pharmaceutically acceptable cations. Preferably, said cation is sodium. Therefore, the composition preferably comprises sodium clodronate.

The composition of the invention therefore comprises crosslinked hyaluronic acid or a salt or derivative thereof. Preferably the crosslinked hyaluronic acid is in the form of a salt, more preferably of an alkali or alkaline earth metal salt, more preferably of sodium. Therefore, the crosslinked hyaluronic acid of the invention is preferably in the form of sodium hyaluronate.

The crosslinked hyaluronic acid, or a salt or derivative thereof, of the invention is a hyaluronic acid subjected to a crosslinking process with a crosslinking agent. Said crosslinking agent is preferably selected from the group consisting of 1,4-butanediol diglycidyl ether (BDDE), divinyl sulfone (DVS) and glutaraldehyde (GTA); more preferably it is 1,4-butanediol diglycidyl ether (BDDE).

The composition of the invention comprises acetate buffer.

According to the invention, the acetate buffer is an aqueous solution of acetic acid and an alkali or alkaline earth metal acetate. Examples of acetate buffer include aqueous solutions of acetic acid-sodium acetate, acetic acid-potassium acetate, acetic acid-calcium acetate, acetic acid-magnesium acetate, and the like. The preferred acetate buffer according to the invention is an aqueous solution of acetic acid-sodium acetate, more preferably an aqueous solution comprising acetic acid, sodium acetate and sodium chloride.

Surprisingly, the presence of the acetate buffer was in fact able to stabilize the composition of crosslinked hyaluronic acid, or a salt or derivative thereof, and clodronic acid, or equivalent amounts of a salt thereof, so effectively that it was possible to also overcome the stability issues connected to the necessary sterilization treatments of the pharmaceutical product during the drug manufacturing phase, which in the absence of acetate buffer, or using other buffers, led instead to rapid degradation of the active clodronic acid/clodronate salt, as will be apparent from the following experimental part.

Clearly, the involved chemical equilibriums among the various species of the composition are complex equilibriums which do not exclusively concern the definition of a pH value that may be capable of stabilizing the species involved, which are however individually notoriously stable at completely different pHs. There are also reasonably other equilibriums and reactions which are also triggered by virtue of the temperature, in particular during the tricky compositions' sterilization phase, which cause chemical reactions and degradations precisely dependent on the chemical species present, regardless of the selected pH value.

In fact, as will be apparent from the experimental part that follows, buffers capable of conferring identical pH values, surprisingly, were not able to ensure the same stability performances to the pharmaceutical compositions prepared during their entire manufacturing cycle, also including the subsequent sterilization step, and not only the preparation of an initially stable solution of crosslinked hyaluronic acid, or a salt or derivative thereof, and clodronic acid or salt thereof, a preparation that is already tricky in itself due to the different stability conditions required by the two active ingredients, but also the subsequent sterilization step.

Downstream of the sterilization phase, in fact, the inventors of the present invention surprisingly discovered that apparently stable compositions, obtained thanks to the use of different buffers which however conferred similar pH values, underwent a different stress, which had an unexpected impact on the stability of these compositions, which showed acceptable impurities profiles and therefore degradation only in the presence of acetate buffer.

Surprisingly, therefore, the acetate buffer plays a fundamental role in the chemistry of these compositions of crosslinked hyaluronic acid, or a salt or derivative thereof, and clodronic acid, or equivalent amounts of a salt thereof, managing to stabilize them, both in the drug formulation phase and in the subsequent sterilization phase of the same, at the same time also ensuring adequate values of *G*', *i.e*. elastic modulus, for their use in the form of a gel to be administered preferably by means of pre-filled syringes, thus allowing their safe and reliable manufacturing and commercialization.

Preferably the acetate buffer of the invention is an aqueous solution of sodium acetate/acetic acid.

The composition of the invention also preferably comprises linear hyaluronic acid or a salt thereof.

The composition of the invention also preferably comprises excipients, more preferably pharmacologically acceptable excipients for intra-articular administration. The composition of the invention may also comprise a pharmaceutically acceptable carrier. This carrier is preferably water.

Suitable pharmaceutically acceptable excipients for intra-articular administration are for example pH regulators, isotonic regulators, stabilizers, chelating agents, preservatives, and antioxidants.

Among the excipients present in the composition, sodium chloride can be mentioned by way of example.

In another aspect, the composition of the invention is in the form of a gel.

In a preferred embodiment said composition, preferably in the form of a gel, comprises:
- from 1.8 to 2.2 mg/mL, preferably from 1.9 to 2.1 mg/mL, more preferably about 2 mg/mL of sodium clodronate or equivalent amounts of clodronic acid or other salt thereof;
- from 16 to 24 mg/mL, preferably from 18 to 22 mg/mL, more preferably about 20 mg/mL of crosslinked sodium hyaluronate;
- from 0.6 to 1.2 mg/mL, preferably from 0.8 to 1 mg/mL of sodium acetate, and from 0.028 to 0.052 mg/mL of acetic acid; and
- Water.

Optionally, the composition of the invention comprises suitable excipients.

In a further preferred embodiment, said composition, preferably in the form of a gel, comprises:
- from 1.8 to 2.2 mg/mL, preferably from 1.9 to 2.1 mg/mL, more preferably about 2 mg/mL di sodio clodronato or equivalent amounts of clodronic acid or other salt thereof,
- from 17 to 21 mg/mL, preferably from 18 to 20 mg/mL, more preferably about 19 mg/mL of crosslinked sodium hyaluronate;
- from 0.5 to 2 mg/mL, preferably about 1 mg/mL of linear sodium hyaluronate;
- from 0.6 to 1.2 mg/mL, preferably from 0.8 to 1 mg/mL, of sodium acetate and from 0.028 to 0.052 mg/mL of acetic acid;
- Water.

Optionally said composition comprises suitable excipients.

The composition of the invention, preferably in the preferred embodiment comprising the addition of linear sodium hyaluronate, advantageously allows to have a gel extrusion force in the range from 18 to 65 N, preferably lower than or equal to 30 N, more preferably of about 30 N, as measured with a universal testing machine at a speed of 1cm/minute; the product is extruded from a 2 mL volume syringe having a 21G1"1/2 type needle.

The obtained composition in the form of a gel was the most preferred for intra-articular administration.

Said composition in the form of a gel could be prepared by a process comprising the following steps:
a. subjecting a linear hyaluronic acid to hydration and crosslinking by means of an aqueous solution comprising a crosslinking agent, obtaining a gel;
b. neutralizing the gel obtained in step (a) by adding a solution of an acid, obtaining a neutralized gel;
c. subjecting the neutralized gel of step (b) to a purification process by means of dialysis, obtaining a dialyzed gel;
d. adding a solution comprising sodium clodronate, acetate buffer and, optionally, a linear hyaluronic acid to the dialyzed gel of step (c);
e. mixing the components of step (d) to obtain a homogeneous gel.

In step (a) the linear hyaluronic acid is hydrated and then crosslinked with an aqueous solution comprising a crosslinking agent, preferably by adding it to the basic hydration solution comprising water and a crosslinking agent. In a preferred form of the invention, the basic aqueous solution of the crosslinking agent is a basic solution of the crosslinking agent, 1,4-butanediol diglycidyl ether (BDDE). Said basic solution comprising 1,4-butanediol diglycidyl ether (BDDE) is preferably a sodium hydroxide aqueous solution.

In step (b) the gel obtained in step (a) is preferably neutralized with a solution of hydrochloric acid diluted in buffer.

In step (d) the acetate buffer is preferably in the form of an aqueous solution of sodium chloride, sodium acetate and acetic acid.

The composition for use of the invention in the form of a gel can advantageously be subjected to further process steps in order to be dispensed with a suitable form of administration.

In a preferred embodiment of the invention, the composition for use in the form of a gel is subjected to a sterilization step following its insertion into an administration form, for example a syringe.

Preferably, the sterilization step takes place in an autoclave at a temperature of 125°C for 250 seconds.

In a preferred embodiment, said composition in the form of a gel has a pH in the range from 4.8 to 5.4, preferably from 5 to 5.2.

In another preferred embodiment, the composition in the form of a gel has a value of the elastic modulus G' at 1 Hz greater than or equal to 50 Pa, measured with a plate rheometer with a gap of 250 µm at 1Hz.

In a further preferred embodiment, the composition for the use of the invention has an osmolarity in the range from 250 to 400 mOsm/L, preferably from 270 to 380 mOsm/L.

In a further preferred embodiment, the composition of the invention in the form of a gel is in a single-dose administration form in a volume from 1 to 5 mL, more preferably 2 mL.

This single-dose administration form is preferably a syringe.

The composition of the invention or the previously described single-dose administration form preferably comprises about 38 mg of crosslinked sodium hyaluronate, about 2 mg of linear sodium hyaluronate, about 4 mg of sodium clodronate, about 1.8 mg of sodium acetate, about 0.08 mg of acetic acid and about 18 mg/mL of sodium chloride.

The composition of the invention or the previously described single-dose administration form is advantageously to be employed for use as a medicament, preferably for use in the treatment of osteoarthrosis.

In another aspect, the composition of the invention can be used alone or in combination, i.e. as a co-therapeutic agent in a fixed dose combination or combination therapy of separately formulated active ingredients, in the treatment of osteoarthrosis.

In yet another aspect, the invention concerns a medical device comprising the composition of the invention and, optionally, suitable excipients.

Said medical device is preferably to be employed for use in improving the symptoms of osteoarthrosis, wherein said improvement comprises a reduction in joint pain. As will be apparent from the experimental part that follows, the composition for the use of the invention is stable, reproducible, and usable for the preparation of single-dose administration forms such as, for example, pre-filled syringes.

The invention is now illustrated by means of some examples to be intended as illustrative and non-limiting of the same.

### EXPERIMENTAL PART

### Example 1. Preparation of a gel composition according to the invention, comprising crosslinked hyaluronic acid, sodium clodronate and acetate buffer.

A gel composition according to the invention was prepared, using the following procedure:
a. a linear hyaluronic acid with an average molecular weight of about 3 MDa was subjected to hydration and crosslinking by adding a basic aqueous solution of sodium hydroxide, at a pH of about 13, comprising 25% of 1,4-butanediol diglycidyl ether (BDDE), until a crosslinked hyaluronic acid gel is formed.
b. the gel obtained in step (a) was then neutralized by adding a solution of hydrochloric acid diluted in acetate buffer;
c. the thus neutralized gel of step (b) was then subjected to a purification process by means of dialysis, obtaining a purified gel characterized by a pH value of about 4.98;
d. an aqueous solution comprising sodium clodronate in a concentration of 100 mg/mL and an aqueous solution of acetate buffer (obtained by mixing 100 mg/mL of acetic acid, 100 mg/mL of sodium acetate and 100 mg/mL of sodium chloride) was added to the dialyzed gel of step (c), and then it was mixed thoroughly until obtaining a homogeneous gel according to the invention.

Said gel exhibited a G' value of about 267.2 Pa, as measured with a plate rheometer with a gap of 250 µm at 1Hz and a pH of about 4.81.

This gel was then subjected to sterilization, *i.e*. to a treatment lasting 250 seconds at a temperature of about 125°C.

At the end of the sterilization treatment, the G' value of the gel dropped to about 77.1 Pa, retaining only about 28.5% of its initial value.

The pH did not undergo appreciable alterations, resulting equal to about 4.88. The sterilized gel was also subjected to accelerated stability tests, at a temperature of 60°C and 96% humidity, and under these stress conditions, the G' value further decreased to about 36.3 Pa.

### Example 2. Preparation of a gel composition comprising crosslinked hyaluronic acid, sodium clodronate and citrate buffer.

A composition identical to the composition of Example 1 was made, with the only difference being the replacement of the acetate buffer of the invention with citrate buffer.

In particular, the added citrate buffer was an aqueous solution comprising 0.98 mg/mL of citric acid, 3.54 mg/mL of sodium citrate and 9 mg/mL of sodium chloride. Also in this case, a crosslinked hyaluronic acid gel was obtained; this gel exhibited a G' value measured with a plate rheometer with a gap of 250 µm at 1Hz equal to about 406.8 Pa and a pH of about 5.0.

The gel was then subjected to sterilization, *i.e*. to a treatment lasting 250 seconds at a temperature of about 125°C.

At the end of the sterilization treatment, the G' value of the gel decreased to about 159.2 Pa, retaining only about 39.0% of its initial value.

The pH did not undergo any alterations and was still equal to about 5.0.

The sterilized gel was also subjected to accelerated stability tests, at a temperature of 60°C and 96% humidity, and under these stress conditions, the G' value further decreased to about 113.1 Pa.

### Example 3. Comparison of the results obtained in the tests carried out on the composition of Example 1 according to the invention and of Example 2. Analysis of the impurity profile of the gel after sterilization.

The results obtained for G' before and after sterilization in the case of the two compositions of Examples 1 (COMP1inv) and 2 (COMP2) were then compared. These results are reported in the following Table 1.

**Table 1 - G' values in the gels of the composition of Example 1 and Example 2, before and after the sterilization step.**

| **Composition** | **Gel G' before sterilization (Pa)** | **Gel G' after sterilization (Pa)** | **% G' value retention** |
|---|---|---|---|
| COMP1inv | 267.2 | 77.1 | 28.5 |
| COMP2 | 406.8 | 159.2 | 39.0 |

As is apparent from the results reported in the table, the composition of Example 2, comprising the citrate buffer, showed an even better profile as regards the parameter G'.

To test the effective possibility of using the two buffers in the gel formulation, the impurity profiles of each gel were analyzed after the sterilization phase, which notoriously can lead to a significant degradation of the active ingredient, in particular of sodium clodronate. The determination of the impurities was carried out by ion chromatography (Thermo Fisher Scientific instrument).

It is known that the impurities related to clodronate and reported in the European Pharmacopoeia (Monograph 1777) consist of the so-called impurity D and phosphate. Surprisingly, in the analyzes carried out by the inventors, impurities not previously described were also found, which were therefore called Unk1, Unk2 and Unk 3 (where the prefix "link" naturally stood for Unknown).

The results obtained by these analyzes (carried out in triplicate, where the mean value is reported) are shown in Table 2 as regards the composition with the citrate buffer of Example 2, and in Table 3 as regards the composition with the acetate buffer of Example 1 according to the invention.

Impurity D was not reported in these tables because it was below the instrument detection threshold in both cases.

**Table 2 - Variation of the clodronate titer in the COMP2 composition following sterilization and its impurities profile.**

| Hyaluronic acid + sodium clodronate + citrate buffer (COMP2) | Clodronate before sterilization (%) | Clodronate after sterilization (%) | Impurities related to clodronate | | | | |
|---|---|---|---|---|---|---|---|
| | | | Phosphate (%) | Unk 1 (%) | Unk 2 (%) | Unk 3 (%) | Tot (%) |
| Mean Value of the three tests | 100.7 | **98.8** | 0.15 | 0.06 | 0.07 | 2.48 | 2.79 |

**Table 3 - Variation of the clodronate titer in the COMP1inv composition following sterilization and its impurities profile.**

| Hyaluronic acid + sodium clodronate + acetate buffer (COMP1inv) | Clodronate before sterilization (%) | Clodronate after sterilization (%) | Impurities related to clodronate | | | | |
|---|---|---|---|---|---|---|---|
| | | | Phosphate (%) | Unk 1 (%) | Unk 2 (%) | Unk 3 (%) | Tot (%) |
| Mean Value of the three tests | 99.3 | **96.2** | 0.51 | 0.14 | 0.33 | 0.97 | **1.94** |

As can be seen from Tables 2 and 3, the results obtained demonstrated the reproducibility and reliability of the method.

In terms of the overall clodronate degradation, the results appeared comparable in the two cases, with the composition of Example 2 comprising the citrate buffer being still slightly better than the results obtained with the acetate buffer of Example 1 according to the invention. In the latter case, in fact, about 1% more of the active was degraded.

However, very surprisingly, what emerged was the totally different profile of the impurities.

Although, in fact, the composition of the two starting gels - except for the buffer - was identical, as well as the pH value of the two gels was almost identical before and after sterilization (about 5 in the case of the citrate buffer, about 4.9 in the case of the acetate buffer according to the invention), completely different values of the individual impurities were obtained.

This is the evidence that chemical-physical equilibriums and chemical reactions are established in the gel which are difficult to predict, and mainly due to the role of the buffer present inside them.

In fact, the chemical species that forms the buffers, in addition to controlling the pH of the composition, in these two cases in the same way, given the same pH values obtained, also influence other properties of the system, *i.e*. the stability of the clodronate in the gel and its tendency to degrade following kinetics which are clearly very different in the two cases.

Therefore, the ability of the citrate buffer and the acetate buffer according to the invention to interact with the hyaluronic acid and clodronate gel are therefore completely different, and clearly this diversity is expressed by the different rheology of the clodronate once placed in contact with one or the other sample, to the point of obtaining such different impurity profiles.

In particular, in the case of the citrate buffer (COMP2, Table 2) the % value of the component called UNK3 was as much as 2.48%.

As known, in the pharmaceutical field, the ICH guidelines advise against the use of active ingredients which, by degrading, could lead to the release of unclassified or unclassifiable substances, in concentrations higher than 1%.

Therefore, the citrate buffer, despite appearing almost like the buffer of choice on the basis of the results obtained for the parameters of G' even under stress conditions, could never be used to formulate compositions of hyaluronic acid and clodronate due to its unsuitable impurities' profile.

The impurity Unk3 in the case of the acetate buffer according to the invention, on the other hand, was detected in a concentration of 0.97%, therefore within the acceptance limits. The same applies to the other two impurities Unk1 and Unk2 both below the identification threshold of 0.5%; with the exception of phosphate, an impurity already known which is not in any case considered a dangerous impurity for human health.

Thanks to the presence of the acetate buffer, the composition of the invention was therefore the only one suitable for pharmaceutical development.

### Example 4. Preparation of a formulation according to the invention further comprising linear hyaluronic acid.

A composition in the form of a gel according to the invention, comprising hyaluronic acid, sodium clodronate, acetate buffer and linear sodium hyaluronate was also prepared.

In the following Table 4 the qualitative-quantitative composition in detail:

**Table 4 - Composition according to the invention in the form of a gel further comprising linear hyaluronic acid.**

| Component | Composition per mL | Composition per syringe (2 mL) |
|---|---|---|
| Crosslinked Sodium Hyaluronate | 19 mg | 38 mg |
| Linear Sodium Hyaluronate | 1 mg | 2 mg |
| Disodium Clodronate | 2 mg | 4 mg |
| Sodium chloride | 9 mg | 18 mg |
| Sodium acetate | 0.9 mg | 1.8 mg |
| Acetic acid | 0.04 mg | 0.08 mg |
| Water for injection | To volume (1mL) | To volume (2 mL) |

The composition of the invention in the form of a gel for intra-articular use shown in Table 4 was prepared according to the same procedure already specified in the previous examples, with the only difference being the addition of linear hyaluronic acid with an average molecular weight of 1.5 MDa to the reaction mixture, in step (d) in conjunction with the addition of the sodium clodronate solution.

The present formulation in gel proved to be perfectly stable over time, even after the sterilization phase, with a level of impurities and a degradation of sodium clodronate totally acceptable according to the Pharmacopoeia standards. Furthermore, its G' value, equal to about 383 Pa, made it easy to administer from a pre-filled syringe, thus allowing the composition to be marketed in a pharmaceutical form with high patient compliance.

## Claims

1. A composition comprising:
- crosslinked hyaluronic acid or a salt or derivate thereof,
- clodronic acid or equivalent amounts of a salt thereof, and
- acetate buffer.

2. The composition according to claim 1, wherein the crosslinked hyaluronic acid or salt or derivate thereof is sodium hyaluronate crosslinked with butanediol diglycidyl ether (BDDE).

3. The composition according to claim 1 or 2, wherein the clodronic acid or salt thereof is sodium clodronate.

4. The composition according to anyone of claims 1 to 3, wherein the acetate buffer is an aqueous solution of acetic acid/sodium acetate, preferably in the form of an aqueous solution of sodium chloride, sodium acetate and acetic acid.

5. The composition according to anyone of claims 1 to 4, wherein said composition is in the form of a gel.

6. The composition according to anyone of claims 1 to 5, wherein said composition comprises:
- from 1.8 to 2.2 mg/mL, preferably from 1.9 to 2.1 mg/mL, more preferably about 2 mg/mL of sodium clodronate or equivalent amounts of clodronic acid or other salt thereof;
- from 16 to 24 mg/mL, preferably from 18 to 22 mg/mL, more preferably about 20 mg/mL of crosslinked sodium hyaluronate;
- from 0.6 to 1.2 mg/mL, preferably from 0.8 to 1 mg/mL of acetate ions and from 0.028 to 0.052 mg/mL of acetic acid; and
- water.

7. The composition according to anyone of claims 1 to 5, wherein said composition comprises:
- from 1.8 to 2.2 mg/mL, preferably from 1.9 to 2.1 mg/mL, more preferably about 2 mg/mL of sodium clodronate or equivalent amounts of clodronic acid or other salt thereof;
- from 17 to 21 mg/mL, preferably from 18 to 20 mg/mL, more preferably about 19 mg/mL of crosslinked sodium hyaluronate;
- from 0.5 to 2 mg/mL, preferably about 1 mg/mL of linear sodium hyaluronate;
- from 0.6 to 1.2 mg/mL, preferably from 0.8 to 1 mg/mL of acetate ions and from 0.028 to 0.052 mg/mL of acetic acid, and
- water.

8. The composition according to anyone of claims 1 to 7, wherein said composition has an extrusion force in the range from 18 to 65 N, preferably lower than or equal to 30 N, more preferably of about 30 N, as measured with a universal testing machine (or universal tester machine) at a speed of 1cm/minute with a 21G1"1/2 needle.

9. The composition according to anyone of claims 1 to 8, wherein said composition has a pH in the range from 4.8 to 5.4, preferably from 5 to 5.2.

10. The composition according to anyone of claims 1 to 9, wherein the composition has a value of the elastic modulus G' at 1 Hz greater than or equal to 50 Pa, as measured with a plate rheometer and a gap of 250 µm at 1Hz.

11. The composition according to anyone of claims 1 to 10, wherein the composition has an osmolarity in the range from 250 to 400 mOsm/L, preferably from 270 to 380 mOsm/L.

12. A single-dose administration form comprising 1 to 5 mL of the composition according to anyone of claims 1 to 11, preferably about 2 mL.

13. The single-dose administration form according to claim 12, wherein said administration form is a syringe.

14. The composition according to anyone of claims 1 to 11, or a single dose administration form according to claim 12 or 13, comprising about 38 mg of crosslinked sodium hyaluronate, about 2 mg of linear sodium hyaluronate for use as a medicament.

15. The composition according to anyone of claims 1 to 11, or a single dose administration form according to claim 12 or 13, for use in the treatment of osteoarthrosis.

16. The composition according to anyone of claims 1 to 11 for use alone or in combination, *i.e*. as a co-therapeutic agent in a fixed dose combination or in a combination therapy of separately formulated active ingredients, in the treatment of osteoarthrosis.

17. A medical device comprising the composition according to anyone of claims 1 to 11 and, optionally, suitable excipients.

18. A medical device comprising the composition according to anyone of claims 1 to 11 for use in the improvement of osteoarthrosis symptoms, wherein said improvement comprises a reduction in joint pain.
